# EUROPEAN PATENT SPECIFICATION

(11) **EP 2 077 902 B1**
(45) Date of publication and mention of the grant of the patent: **23.03.2016**
(21) Application number: 07747386.6
(22) Date of filing: 19.04.2007
(51) Int. Cl.: A23L 33/10, A23L 9/20, A61K 31/201, A61P 3/00, A61K 45/06, A61K 31/202, A61K 31/70

(54) **USE OF NUTRITIONAL COMPOSITIONS FOR PREVENTING VISCERAL ADIPOSITY**
VERWENDUNG VON ERNÄHRUNGSZUSAMMENSETZUNGEN ZUR VERHINDERUNG DER VISZERALEN ADIPOSITÄT
UTILISATION DE COMPOSITIONS NUTRITIVES POUR EMPÊCHER L'ADIPOSITÉ VISCERALE

(30) Priority: 02.11.2006 WO PCT/NL2006/050274
(43) Date of publication of application: 15.07.2009
(60) Divisional application: 16150456.8
(73) Proprietor: N.V. Nutricia, 2712 HM Zoetermeer (NL)
(72) Inventor: ZWIJSEN, Renate Maria Louise, 3588 VE Utrecht (NL); VAN DER BEEK, Eline Marleen, 6705 DB Wageningen (NL); SPEELMANS, Gelske, 6708 LW Wageningen (NL); BOEHM, Günther, 61209 Echzell (DE)
(74) Representative: Nederlandsch Octrooibureau
(86) International application number: PCT/NL2007/050162
(87) International publication number: WO 2008/054200

(56) References cited:
- EP-A- 1 557 096
- WO-A-01/78530
- WO-A-2005/051091
- WO-A-2006/057551
- WO-A-2006/091103
- WO-A-2007/073192
- WO-A-2007/118073
- WO-A-2008/054192
- AILHAUD ET AL: "Temporal changes in dietary fats: Role of n-6 polyunsaturated fatty acids in excessive adipose tissue development and relationship to obesity" PROGRESS IN LIPID RESEARCH, PERGAMON PRESS, PARIS, FR, vol. 45, no. 3, 1 May 2006 (2006-05-01), pages 203-236, XP005399131 ISSN: 0163-7827
- STOLK R P ET AL: "Validity and reproducibility of ultrasonography for the measurement of intra-abdominal adipose tissue" INTERNATIONAL JOURNAL OF OBESITY, vol. 25, no. 9, September 2001 (2001-09), pages 1346-1351, XP002519006 ISSN: 0307-0565 cited in the application
- SUZUKI R ET AL: "Abdominal wall fat index, estimated by ultrasonography, for assessment of the ratio of visceral fat to subcutaneous fat in the abdomen" AMERICAN JOURNAL OF MEDICINE, XX, XX, vol. 95, no. 3, 1 September 1993 (1993-09-01), pages 309-314, XP023306348 ISSN: 0002-9343 [retrieved on 1993-09-01] cited in the application
- WAJCHENBERG B L: "Subcutaneous and visceral adipose tissue: their relation to the metabolic syndrome." ENDOCRINE REVIEWS DEC 2000, vol. 21, no. 6, December 2000 (2000-12), pages 697-738, XP002519010 ISSN: 0163-769X
- R. P. Tracy: "Is Visceral Adiposity the "Enemy Within"?", Arteriosclerosis, thrombosis, and vascular biology, vol. 21, no. 6, 1 June 2001 (2001-06-01), pages 881-883, XP055207612, ISSN: 1079-5642, DOI: 10.1161/01.ATV.21.6.881

## Description

### FIELD OF THE INVENTION

The present invention relates to prevention of visceral adiposity and of diseases later in life by administering particular nutritional formulae to infants.

### BACKGROUND OF THE INVENTION

Breast-feeding is the preferred method of feeding infants. Breast feeding early in life may influence the occurrence of disorders later in life. However, there are circumstances that make breast-feeding impossible or less desirable. In those cases infant formula and follow-on formula are a good alternative. The composition of modem infant or follow-on formulas is adapted in such a way that it meets many of the special nutritional requirements of the fast growing and developing infant.

Still it seems that improvements can be made towards the constitution of infant milk formula. For example little is known about the effects of ingredients in the infant formula on visceral adiposity and/or health later in life. The present invention relates to such future health.

WO 2006069918 describes a method of continuously reducing the circulating level of insulin like growth factor 1 (IGF-1) in the first few months of the life of an infant by administering to the infant a nutritional composition comprising proteins in an amount such that the composition contains less than 2.25g of protein per 100kcal. As IGF-1 is known to be a key control point in nutritional regulation of growth, this may offer a method of reducing the risk of developing obesity later life. WO 2007004878 relates to a method to treat and/or prevent childhood obesity comprising administering a nutritional composition containing fat, digestible carbohydrates and protein, wherein the protein comprises at least 25 wt.% peptides with a chain length of 25 to 30 amino acids based on dry weight of protein.

### SUMMARY OF THE INVENTION

The present inventors have recognized that the total adipose tissue of infants is not a good predictor to determine the risks of diseases later in life. Body fat can be distributed and stored in fat tissue at different locations within the body. Different fat tissues have different metabolic effects, particularly in infants. Subcutaneous fat tissue in infants has the important function to maintain an adequate body temperature. Fat tissue deposited in the central part of the body (visceral fat) serves only as storage of energy. Furthermore, adipose cells at different locations differ in size and in their protein secretion profile that are potential regulators of glucose and lipid homeostasis. Importantly, visceral fat tissue is a highly metabolically active tissue that releases free fatty acids directly into the hepatic portal vein. The obtained free fatty acid fluxes have an impact on glucose metabolism and insulin sensitivity of the liver and subsequently lead to metabolic disorders. Therefore, a main contributor of the development of certain disorders later in life is the distribution of fat tissue, particularly in case of an imbalanced ratio between visceral and subcutaneous fat tissue and/or excessive visceral fat tissue development in early infancy. Visceral fat tissue accumulation, and in particularly an increased ratio of visceral fat/subcutaneous fat tissue, was found to be a main contributor of development disorders, particularly of metabolic and cardiovascular disorders, independent of overall obesity.

For adipose cell proliferation two critical windows have been determined: during late gestation/early infancy and to a lesser extend during puberty. Outside these time frames cell numbers remain approximately the same and persist throughout adulthood. Hence, it is highly desirable to direct in early infancy the distribution of body fat towards a relative low number of visceral fat cells compared to the number of subcutaneous fat cells. A relative low visceral adipocyte count prevents large amounts of visceral adipose tissue and subsequently prevents metabolic disorders later in life.

Hence, it is the aim of the present invention to prevent disorders later in life and/or to reduce visceral fat adipocyte formation in infancy and/or to reduce the ratio of visceral fat/subcutaneous fat tissue area in infancy.

Finding adequate measures to modify visceral adipocyte proliferation in infants is particularly difficult, as administration of pharmaceutical compounds is generally unacceptable and nutrition cannot be rigorously modified because the infant needs to receive sufficient nutrients for normal growth and development of e.g. organs, muscles and brain. Hence, a main aim of the present invention is to reduce the development of excessive visceral fat tissue accumulation in infancy and/or later in life by designing a nutrition to be administered to the infant which ensures maintenance of normal growth and development.

So far, research on factors influencing body fat distribution in early life has been hampered by a lack of safe and easy accessible techniques to measure visceral adipose tissue in infants. Therefore, the inventors developed a reliable method to determine by ultrasonography visceral and subcutaneous fat tissue distribution in children from 12 months of age up to 4 years.

Unexpectedly, using this method, the inventors found already diet-dependent effects on the ratio visceral/subcutaneous fat tissue in infants at 13 months of age. It was surprisingly found that administration of a nutritional composition low in protein concentration resulted in a reduced ratio of visceral/subcutaneous fat tissue without effecting the body weight, body length and body mass index. This decreased ratio is considered to be predictive for an increased risk of visceral adiposity later in life and/or for increased risk on disorders later in life. Administration of nutritional compositions low in protein enables early intervention in the diet to reduce the risk on disorders later in life.

It was found by the inventors that administration of a composition with a reduced protein concentration, at comparable isocaloric intake, in particular reduces postprandial insulin kinetics (insulin peak and area under the curve (AUC)), and to a lesser extent the postprandial glucose kinetics (glucose peak and AUC), which finally may result in a reduced insulin resistance (or increased insulin sensitivity). Among the different adipose tissues, the visceral adipocytes are the most responsive for these signaling molecules (insulin and glucose) by having more relevant membrane receptors, resulting in an increased uptake of glucose into the fat cell, conversion to fat and, when the critical mass has been achieved, proliferation of the fat cell. In this way, insulin is an important factor of specific proliferation of visceral adipocytes. Hence, a reduced insulin peak and AUC and reduced insulin resistance and/or insensitivity in infancy is indicative for the prevention of visceral adiposity and/or for the prevention of diseases later in life.

### DETAILED DESCRIPTION OF THE INVENTION

In one embodiment the present invention concerns the use of a composition comprising a lipid, protein and digestible carbohydrate component wherein the protein component provides less than 9% of the total calories, the lipid component provides 35 to 55% of the total calories and the digestible carbohydrate component provides 30 to 60% of the total calories for the manufacture of a nutritional composition to be administered to a human with an age below 36 months and for preventing and/or treating visceral adiposity; b) preventing and/or treating the accumulation of visceral fat tissue; and/or c) decrease the ratio of visceral fat to subcutaneous fat tissue area.

In one embodiment the present invention concerns the use of a composition comprising a lipid, protein and digestible carbohydrate component wherein the protein component provides less than 9% of the total calories, the lipid component provides 35 to 55% of the total calories and the digestible carbohydrate component provides 30 to 60% of the total calories for the manufacture of a nutritional composition to be administered to a human with an age below 36 months and for preventing visceral adiposity at the age above 36 months, particularly to prevent visceral adiposity at the age above 8 years, particularly above 13 years.

### Visceral adiposity

Visceral adiposity differs from general adiposity or general obesity. Human subjects may suffer from general obesity due to increased subcutaneous fat deposits, but not suffer from visceral adiposity. On the other hand, human subjects may suffer from visceral adiposity, without suffering from general obesity. The increased risk of health problems later in life, such as diabetes and cardiovascular diseases, is related to the occurrence of visceral adiposity and not to general obesity.
The term 'visceral adiposity' refers to a condition with increased visceral fat tissue. Visceral adiposity is typically caused by (accumulation of) excessive visceral fat tissue. Visceral fat, also known as organ fat, intra-abdominal fat, peritoneal fat or central fat, is normally located inside the peritoneal cavity as opposed to subcutaneous fat which is found underneath the skin and intramuscular fat which is found interspersed in skeletal muscles. Visceral fat includes the abdominal fat surrounding the vital organs and includes mesenteric fat, perirenal fat, retroperitoneal fat and preperitoneal fat (fat surrounding the liver). In humans beyond infancy, visceral adiposity is also referred to as central obesity, 'apple-shaped' obesity, 'android' obesity, 'abdominal' obesity, 'male-type' obesity, 'waist-predominant' obesity, 'truncal' obesity or 'masculine' obesity. A waist circumference above 102 cm in adult man or above 88 cm in adult women indicates the presence of visceral adiposity. Also a waist-hip ratio can be used as indicator for visceral adiposity. Hip-waist ratio's exceeding 0.9 in man and 0.85 in women indicate visceral adiposity. For children of 3-19 years old appropriate cutoffs for age- and sex-dependent waist circumferences can be found in Taylor et al, 2000 Am J Clin Nutr 72:490-495. Visceral fat stores in adults and children can be investigated by imaging techniques including computed tomography (CT), and magnetic resonance imaging (MRI). These methods are accurate imaging techniques for children below 4 years but the disadvantages are costs, radiation exposure (for CT) and the use is very limited to a research setting. Ultrasonography (US) has been proposed as an alternative non-invasive technique to measure subcutaneous and visceral fat stores, because it overcomes these disadvantages. However, for children up to 4 years so far no suitable ultrasound imaging techniques are available.
An adult is considered to suffer from visceral adiposity or to have accumulated visceral fat tissue to an excessive amount when at the umbilicus level the visceral adipose tissue (VAT) exceeds 100 cm² in man, or 90 cm² in women (Saito et al, 1009, Int J Obes Suppl 3:S226) as determined with imaging techniques.
A subject suffers from visceral adiposity when it meets one or more of the above criteria (regarding VAT, waist circumference or waist-hip ratio thresholds). Accumulation of visceral fat tissue to an excessive amount relates to the accumulation of visceral fat tissue to a level at which visceral adiposity occurs and can be determined by the same methods as described above for visceral adiposity.

A non-obese human below 36 months of age has gender specific body mass index (BMI)-for age below the 95^{th} percentile, more preferably below the 85^{th} percentile. BMI is an anthropometric measure defined as weight in kilograms divided by the square of length in meters. Tables with gender specific BMI-for age are publicly available for instance at the US National Center for Health Statistics.

### Method to determine visceral adiposity in human subjects below 48 months of age

Besides the highly expensive, patient-unfriendly and time-consuming MRI and CT methods, at present there are no suitable methods to determine visceral adiposity in children below 4 years. As already mentioned, ultrasonography (US) methods to detect visceral fat versus subcutaneous fat are not suitable for infants and young children below 4 years. This is due to several reasons, such as that the measurement is not sensitive enough (the visceral fat layers in infants are very thin: less than 10% of the total body fat), and the different morphology of immature and developing organs. The most often used US method in adults has been described by Armellini F., Basic Life Sci. 60:75-7 and Stolk RP, 2001, Int J Obes Relat Metab Disord 25:1346-51. This is an indirect method of fat layers measuring the intra-abdominal fat (IAM) distance: the distance between the peritoneum and 1. the (dorsal side of) the aorta and/or 2. the spine. In this area, central fat layers have been found, although it also includes all tissue that is present in the abdomen (e.g. intestine). This method has been applied by the inventors to children of 9, 12, 24, and 36 months and 6 years old by placing the electronic calipers longitudinally at the height of the navel. It was found that in infants below 36 months, particularly below 24 months, and more particularly in the first 12 months, the outcome was influenced by many confounders, such as that the intestine can be filled with varying amounts of air, varying size of abdomen, varying stool properties, varying muscle contraction status. Therefore, this method does not lead to a realistic estimation of visceral fat and fat distribution and is not suitable for human subjects below 48 months.

An ultrasonography method for adults as described by Suzuki et al, 1993, Am J Med 95:309-14, was adapted in order to make this method suitable for human subjects below 48 months of age. In both the present method and the Suzuki method, scanning was longitudinally from the xiphoid process to the navel along the midline (linea alba).
After intensive research, the following adaptations were found to be essential: 1) adaptation relating to the precise location of measurement, and 2) adaptation relating to the unit of measurement.
Ad 1: In the method of Suzuki, 1993, the reference point of the preperitoneal fat layer was determined by the maximal thickness and the reference point of the subcutaneous fat layer was determined by the minimal thickness. In the method according to the present invention, using (very) young children, the optimal image to measure with regard to midline cut and parallelity of the fat layers was chosen. Therefore, as the apical reference point the point was taken where the slope of the different fat layers (i.e. the subcutaneous fat layer and the preperitoneal fat layer) changes and the different fat layers start to be situated in parallel to each other.
Ad 2: In the present method areas instead of distances were measured in order to increase sensitivity of the method. It was found that measurement of preperitoneal fat in children below 48 months of age as the area of fat tissue 1 cm or 2 cm along the midline, starting from the reference point in direction of the navel, gave more reliable results than measurement of the distance of the linea alba to the peritoneum on top of the liver as in the Suzuki method. More remarkably, in the very young children with an age below 24 months, measurement of the area between 1 cm and 2 cm along the midline gave even better results. It was also found that measurements of subcutaneous fat in children below 48 months of age as the area of fat tissue of 1 cm or 2 cm length along the midline, starting from the reference point in direction of the navel, gave more reliable results than measurement of distance of the inner surface of subcutaneous tissue to the linea alba as in the Suzuki method. Remarkably also here, in the very young children with an age below 24 months, measurement of the area between 1 cm and 2 cm along the midline gave even better results than measurement of distances.

### Protein

Protein is present in the composition below 9% based on calories. Preferably the nutritional composition comprises between 7.2 and 8.0% based on total calories, more preferably between 7.3 and 7.7% based on total calories. As total calories of the composition the sum of calories delivered by the fats, proteins and digestible carbohydrates of the composition is taken. A low protein concentration ensures a lower insulin response, thereby preventing proliferation of adipocytes, especially visceral adipocytes in infants. The protein concentration in a nutritional composition is determined by the sum of protein, peptides and free amino acids. The protein concentration is determined by determining the amount of Nitrogen, multiplying this with a factor 6.38. One gram of protein equals 4 kcal. Based on dry weight the composition preferably comprises less than 12 wt.% protein, more preferably between 9.6 to 12 wt.%, even more preferably 10 to 11 wt.%. Based on a ready-to-drink liquid product the composition preferably comprises less than 1.5 g protein per 100 ml, more preferably between 1.2 and 1.5 g, even more preferably between 1.25 and 1.35 g.
The source of the protein should be selected in such a way that the minimum requirements for essential amino acid content are met and satisfactory growth is ensured. Hence protein sources based on cows' milk proteins such as whey, casein and mixtures thereof and proteins based on soy are preferred. In case whey proteins are used, the protein source is preferably based on acid whey or sweet whey, whey protein isolate or mixtures thereof and may include α-lactalbumin and β-lactoglobulin. More preferably, the protein source is based on acid whey or sweet whey from which the caseino-glyco-macropeptide (CGMP) has been removed. Removal of CGMP from sweet whey protein or the use of acid whey advantageously reduces the threonine content of the sweet whey protein. Optionally the protein source may be supplemented with free amino acids, such as methionine, histidine, tyrosine, arginine and tryptophan in order to improve the amino acid profile. Preferably α-lactalbumin enriched whey protein is used in order to optimize the amino acid profile. Using protein sources with an optimized amino acid profile closer to that of human breast milk enables all essential amino acids to be provided at reduced protein concentration, below 9 % based on calories, preferably between 7.2 and 8.0% based on calories and still ensure a satisfactory growth.
If modified sweet whey is used as the protein source, it is preferably supplemented by free arginine in an amount of from 0.1 to 3 wt.% and/or free histidine in an amount of from 0.1 to 1.5 wt.% based on total protein.

The proteins may be intact or hydrolysed or a mixture of intact and hydrolysed proteins although intact proteins are generally preferred. Preferably the composition comprises hydrolyzed casein and/or hydrolyzed whey protein. It was found that administration of a composition wherein the protein comprises hydrolyzed casein and hydrolyzed whey results in reduced post-prandial levels of both insulin and glucose compared to the administration of a composition comprising intact casein and intact whey protein. Increased levels of both insulin and glucose indicate a form of insulin insensitivity and/or resistance in formula fed infants. The present composition preferably comprises at least 25 wt.% peptides with a chain length of 2 to 30 amino acids based on dry weight of protein. The amount of peptides with a chain length between 2 and 30 amino acids can for example be determined as described by de Freitas et al, 1993, J. Agric. Food Chem. 41:1432-1438. The present composition may include casein hydrolysate or the present composition may include whey protein hydrolysate. The present composition preferably includes both casein hydrolysate and whey protein hydrolysate because the amino acid composition of bovine casein is more similar to the amino acid composition found in human milk protein and whey protein is easier to digest and found in greater ratios in human milk. The composition preferably comprises at least 50 wt.%, preferably at least 80 wt.%, most preferably about 100 wt.% of a protein hydrolysate, based on total weight of the protein. The present composition preferably comprises a protein with a degree of hydrolysis of the protein between 5 and 25%, more preferably between 7.5 and 21%, most preferably between 10 and 20%. The degree of hydrolysis is defined as the percentage of peptide bonds which have been broken down by enzymatic hydrolysis, with 100% being the total potential peptide bonds present. A suitable way to prepare a hydrolysate is described in WO 01/41581.

Casein is advantageously present since it increases the gastric emptying times by forming a curd in the stomach, thereby increasing satiety. Preferably the composition comprises at least 3 wt.% casein based on dry weight. Preferably the casein is intact and/or non-hydrolyzed.

### Lipid component

Herein LA refers to linoleic acid (18:2 n6); ALA refers to alpha-linolenic acid (18:3 n3): LC-PUFA refers to long chain polyunsaturated fatty acids comprising at least 20 carbon atoms in the fatty acid chain and with 2 or more unsaturated bonds; DHA refers to docosahexaenoic acid (22:6, n3); EPA refers to eicosapentaenoic acid (20:5 n3): ARA refers to arachidonic acid (20:4 n6); Medium chain fatty acids (MCFA) are fatty acids and/or acyl chains with a chain length of 6, 8 or 10 carbon atoms.

When in liquid form, e.g. as a ready-to-feed liquid, the composition preferably comprises 2.1 to 6.5 g fat per 100 ml, more preferably 3.0 to 4.0 g per 100 ml. Based on dry weight the present composition preferably comprises 12.5 to 40 wt.% fat, more preferably 19 to 30 wt.%. The present composition preferably comprises a lipid, component providing 35 to 55% of the total calories, more preferably 40 to 50% of the total calories.

Compositions that have a low LA/ALA ratio and/or that are low in LA prevent the occurrence of visceral adiposity. Particularly the administration of a nutritional composition comprising a LA/ALA ratio between 2 and 7 and/or a low LA content (<15 wt.% based on total fatty acids) results in a decrease in visceral adiposity later in life and/or a reduced occurrence of disorders later in life. MCFA reduce adiposity in general. Therefore, an optimal composition additionally preferably comprises MCFA, but not in excessive amounts, i.e. between 3 and 50 wt.% based on weight of total fatty acids.

LA should preferably be present in a sufficient amount in order to promote a healthy growth and development, yet in an amount as low as possible to prevent occurrence of visceral adiposity. The composition therefore preferably comprises less than 15 wt.% LA based on total fatty acids, preferably between 5 and 14.5 wt.%, more preferably between 6 and 12 wt.%.

The weight ratio LA/ALA should preferably be well balanced in order to prevent visceral fat tissue deposition (e.g. visceral adiposity) and disorders later in life, while at the same time ensure a normal growth and development. The present composition preferably comprises a weight ratio of LA/ALA between 2 and 7, more preferably between 3 and 6, even more preferably between 4 and 5.5, even more preferably between 4 and 5. The lipid component preferably comprises less than 15 wt.% LA based on total fatty acids and a LA/ALA ratio of 2 to 7.

Preferably the present composition comprises n-3 LC-PUFA. n-3 LC-PUFA prevents the occurrence of visceral adiposity. More preferably, the present composition comprises n-3 LC-PUFA, even more preferably EPA, DPA and/or DHA. It was found that these LC-PUFA decrease the visceral adiposity.

Since a low concentration of DHA, DPA and/or EPA is already effective and normal growth and development are important, the content of LC-PUFA in the present composition, preferably does not exceed 15 wt.% based on total fatty acids, preferably does not exceed 10 wt.%, even more preferably does not exceed 5 wt.%. Preferably the present composition comprises at least 0.1 wt.%, preferably at least 0.25 wt.%, more preferably at least 0.5 wt.%, even more preferably at least 0.75 wt.% LC-PUFA based on total fatty acids. For the same reason, the EPA content preferably does not exceed 5 wt.% based on total fatty acids, more preferably does not exceed 1 wt.%, but is preferably at least 0.025 wt.%, more preferably at least 0.05 wt.% based on total fatty acids. The DHA content preferably does not exceed 5 wt.%, more preferably does not exceed 1 wt.%, but is at least 0.1 wt.% based on total fatty acids. The DPA content preferably does not exceed 1 wt.%, more preferably does not exceed 0.5 wt.% of the total fat, but is at least 0.01 wt.% based on total fatty acids.
As ARA counteracts the effect of n-3 LC-PUFA on visceral adiposity, the present composition comprises relatively low amounts or ARA. The ARA content preferably does not exceed 5 wt.%, more preferably does not exceed 1 wt.%, more preferably; does not exceed 0.5 wt.%, even more preferably does not exceed 0.25 wt.%, most preferably does not exceed 0.05 wt.% based on total fatty acids. The LC-PUFA, LA, ALA, ARA etc may be provided as free fatty acids, in triglyceride form, in diglyceride form, in monoglyceride form, in phospholipid form, or as a mixture of one of more of the above, preferably in triglyceride and/or phospholipid form.

Suitable lipid sources to be mixed in order to obtain the fat blend of the invention are linseed oil (flaxseed oil), rape seed oil (including colza oil, low erucic acid rape seed oil and canola oil), salvia oil, perilla oil, purslane oil, lingonberry oil, sea buckthorn oil, hemp oil, high oleic sunflower oil, high oleic safflower oil, olive oil, marine oils, microbial oils, black currant seed oil, echium oil, butter fat, coconut oil, and palm kernel oil. Preferred oil sources are selected from the group consisting of linseed oil, rapeseed oil, coconut oil, high oleic sunflower oil, butter oil and marine oil.

### Lactose

The maintenance of insulin sensitivity can be further improved by inclusion of a low glycaemic digestible carbohydrate in the present composition, preferably lactose. Hence, the present composition preferably comprises in addition to the present lipid component, non-digestible oligosaccharides and/or lactose. The present composition preferably comprises a digestible carbohydrate component, wherein at least 35 wt.%, more preferably at least 50 wt.%, more preferably at least 75 wt.%, even more preferably at least 90 wt.%, most preferably at least 95 wt.% is lactose. The present composition preferably comprises at least 25 grams lactose per 100 gram dry weight of the present composition, preferably at least 40 grams lactose/100 gram. The present composition preferably comprises a digestible carbohydrate component providing 30 to 60% of the total calories, more preferably 40 to 50% of the total calories.

### Non-digestible oligosaccharides

It was found that non-digestible oligosaccharides (NDO) that can be fermented (particularly galacto-oligosaccharides) have a blood insulin tempering effect, and consequently contributes to a reduced proliferation of visceral adipocytes. Therefore, the present composition preferably comprises the present low protein component and a non-digestible oligosaccharide which can be fermented. The combination of the present low protein component and the non-digestible oligosaccharides synergistically reduces the visceral adiposity and/or prevents the development of disorders later in life.

Preferably the non-digestible oligosaccharides have a DP between 2 and 60. The non-digestible oligosaccharide is preferably selected from the group consisting of fructo-oligosaccharides (including inulin), galacto-oligosaccharides (including transgalacto-oligosaccharides), gluco-oligosaccharides (including gentio-, nigero- and cyclodextrin-oligosaccharides), arabino-oligosaccharides, mannan-oligosaccharides, xylo-oligosaccharides, fuco-oligosaccharides, arabinogalacto-oligosaccharides, glucomanno-oligosaccharides, galactomanno-oligosaccharides, sialic acid comprising oligosaccharides and uronic acid oligosaccharides. Preferably the present composition comprises fructo-oligosaccharides, galacto-oligosaccharides and/or galacturonic acid oligosaccharides, more preferably galacto-oligosaccharides, most preferably β-linked galacto-oligosaccharides. In a preferred embodiment the composition comprises a mixture of β-linked galacto-oligosaccharides and fructo-oligosaccharides, more preferably in a weight ratio of 20-2 : 1 more preferably 12-7 : 1. Preferably the present composition comprises galacto-oligosaccharides with a DP of 2-10 and/or fructooligosaccharides with a DP of 2-60. The galacto-oligosaccharide is preferably selected from the group consisting of β-linked galaco-oligosaccharides, transgalacto-oligosaccharides, galacto-oligosaccharides, lacto-N-tetraose (LNT), lacto-N-neotetraose (neo-LNT), fucosyl-lactose, fucosylated LNT and fucosylated neo-LNT. β-linked galacto-oligosaccharides are for example sold under the trademark Vivinal™ (Borculo Domo Ingredients, Netherlands). Preferably the saccharides of the galacto-oligosaccharides are β-linked, since this is also the case in human milk galacto-oligosaccharides. Fructo-oligosaccharide is a NDO comprising a chain of β linked fructose units with a DP or average DP of 2 to 250, more preferably 10 to 100. Fructo-oligosaccharide includes inulin, levan and/or a mixed type of polyfructan. An especially preferred fructo-oligosaccharide is inulin. Fructo-oligosaccharide suitable for use in the compositions is also already commercially available, e.g. Raftiline®HP (Orafti). Uronic acid oligosaccharides are preferably obtained from pectin degradation, more preferably apple pectin, beet pectin and/or citrus pectin. Preferably the composition comprises β-linked galacto-oligosaccharide: fructo-oligosaccharide: uronic acid oligosaccharide in a weight ratio of 20-2 : 1 : 1-3 more preferably 12-7 : 1 : 1-2.

### Nutritional composition

The present composition is particularly suitable for providing the daily nutritional requirements to a human subject with an age below 36 months, particularly a human subject with an age below 24 months, even more preferably an infant with an age of 0 to 12 months. Hence, the present composition comprises a lipid, protein and digestible carbohydrate component wherein the lipid component provides 35 to 55% of the total calories, the protein component provides up to 9% of the total calories and the digestible carbohydrate component provides 30 to 60% of the total calories. Preferably the present composition comprises a lipid component providing 40 to 50 % of the total calories, the protein component provides 7.2 to 8% of the total calories and the digestible carbohydrate component provides 40 to 50% of the total calories.

The present composition is not human breast milk. The present composition preferably comprises (i) vegetable lipid and/or animal (non-human) fat; and/or (ii) vegetable protein and/or animal (non-human) milk protein. Examples of animal milk protein are whey protein from cow's milk and protein from goat milk. Preferably the present composition does not comprise a proteinase inhibitor, preferably not a trypsin inhibitor, chymotrypsin inhibitor or elastase inhibitor. The absence of a protease inhibitor ensures a sufficient bioavailibity of protein which is especially important at low protein levels.

The present composition is preferably administered in liquid form. In order to meet the caloric requirements of the the human subject with the age below 36 months, preferably an infant, the composition preferably comprises 50 to 200 kcal/100 ml liquid, more preferably 60 to 90 kcal/100 ml liquid, even more preferably 60 to 75 kcal/100 ml liquid. This caloric density ensures an optimal ratio between water and caloric consumption. The osmolarity of the present composition is preferably between 150 and 420 mOsmol/l, more preferably 260 to 320 mOsmol/l. The low osmolarity aims to reduce the gastrointestinal stress. Stress can induce adipocyte formation.

Preferably the composition is in a liquid form, with a viscosity between 1 and 60 mPa.s. more preferably between 1 and 20 mPa.s, even more preferably between 1 and 6 mPa.s The viscosity of the liquid is determined using a Physica Rheometer MCR 300 (Physica Messtechnik GmbH, Ostfilden, Germany) at shear rate of 95 s⁻¹ at 20 °C. The low viscosity ensures a proper administration of the liquid, e.g. a proper passage through the hole of a teat. Also this viscosity closely resembles the viscosity of human milk. The prevent composition is preferably prepared by admixing a powdered composition comprising with water. Normally infant formula is prepared in such way. The present invention thus also relates to a packaged power composition wherein said package is provided with instruction to admix the powder with a suitable amount of liquid, thereby resulting in a liquid composition with a viscosity between 1 and 60 mPa.s.

When the composition is a liquid form, the preferred volume administered on a daily basis is in the range of about 80 to 2500 ml, more preferably about 450 to 1000 ml per day.

### Human subjects below 48 months of age

Since the most important period to establish the number of visceral adipocytes are the first 36 months of life the present composition is administered to a human subject during the first 3 years of life. Since there is a predominance of proliferation of visceral adipocytes in the first 12 months of life it is particularly important that the present composition is administered to an infant in this period of life. The present composition is therefore advantageously administered to a human of 0-24 months, more preferably to a human of 0-18 months, most preferably to a human of 0-12 months. The present invention particularly aims to prevent disease development later in life and is in one embodiment not a curative treatment. Hence, the present composition is in one embodiment administered to a human subject below 48 months of age not suffering from obesity or childhood obesity, preferably a non-obese infant, more preferably an infant that does not suffer from overweight. The present composition is preferably administered orally to the human subject below 48 months of age. In another embodiment the present composition is administered to a human subject below 48 months of age, preferably an infant, having an aberrant, in particular higher, ratio of visceral/subcutaneous fat tissue area compared to the ratio visceral/subcutaneous fat tissue area observed in breast fed human subjects below 48 monts of age, preferably infants.

### Application

The composition aims to a) prevent and/or treat visceral adiposity, b) prevent and/or treat the accumulation of excessive visceral fat tissue and/or c) increase the ratio subcutaneous/visceral fat tissue in human subjects below 48 months of age. Accumulation of excessive visceral fat tissue means accumulation of the visceral fat tissue exceeding that of visceral fat tissue accumulation observed in breast fed infants. Suitably the visceral fat tissue can be determined by the ultrasonography method according to the present invention. In one embodiment excessive accumulation of visceral fat tissue means accumulation to a value of more than two times standard deviation of the mean and/or median value of visceral fat tissue of a representative group of exclusively breast-fed infants in Western-Europe. In one embodiment the present composition aims to prevent and/or treat the accumulation of visceral fat tissue to an excessive amount.

Decreasing the ratio visceral/subcutaneous fat tissue means adjusting this ratio towards a level that is observed in breast fed infants. Suitably the visceral fat tissue or the ratio visceral/subcutaneous fat tissue can be determined by the ultrasonography method according to the present invention. For example, an unhealthy ratio of visceral/subcutaneous fat tissue in infants using the new US method is a ratio exceeding 1.0 in humans of 13 months of age. By administering the present composition to an infant having such an unhealthy ratio, the ratio visceral/subcutaneous fat tissue is decreased towards the mean and/or median of a representative group of exclusively breast-fed infants in Western-Europe. Particularly, the present invention relates to a method for preventing and/or treating visceral adiposity and/or decrease the ratio visceral/subcutaneous fat tissue in a human subject with the age below 36 months. The present invention also aims to prevent visceral adiposity at the age above 36 months, particularly to prevent visceral adiposity at the age above 8 years, particularly above 13 years. The present inventors found that the present reduction in visceral adiposity suitably reduces occurrence and prevalence of disorders later in life, particularly disorders linked to visceral adiposity. The present invention also provides a method for preventing the development of a disorder in a human with an age above 36 months, wherein said disorder is selected from the group consisting of diabetes (particularly type 2 diabetes), fasting hyperglycaemia, insulin resistance, hyperinsulinemia, hypertension, cardiovascular disease, visceral adiposity, cerebrovascular disease, artherosclerose, dyslipidaemia, hyperuricaemia, fatty liver, osteoarthritis and sleep apnoea, wherein the method comprises the administration of the present composition to a human subject with an age below 36 months. The term dyslipidaemia includes the following diseases: hyperlipidaemia, hyperlipoproteinaemia, hyperchylomicronaemia, hypercholesteraemia, hypoalphalipoproteinemia hypoHDL/LDL-aemia and hypertriglyceridaemia. Particularly the development of diabetes, visceral adipocity and/or cardiovascular diseases can be prevented, more preferably cardiovascular diseases. The present method is particularly suitable to prevent the abovementioned disorders during adolescence, in particular from 13 - 18 years and/or adulthood, in particular above 18 years.
The present invention preferably relates to a method form treating and/or preventing of a disorder selected from the group consisting of type 2 diabetes, fasting hyperglycaemia, insulin resistance and/or insensitivity, and hyperinsulinemia in a human subject with an age below 36 months.

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one".

### Example 1: Low protein beneficially affects insulin sensitivity

*Protein preparations:* Feeding 1a comprised 1.7 g protein per 100 ml and 9.6 g lactose per 100 ml. The protein was composed of 40 wt.% casein and 60 wt.% whey protein. Feeding 1b comprised 1.2 g protein per 100 ml at a ratio of 40 wt.% hydrolyzed casein and 60 wt.% hydrolyzed whey and 9.6 g lactose per 100 ml. Feeding 2a and 2b were volume matched compositions of human milk and infant formula (IMF). Feeding 2a, human milk, comprised per 100 ml 65 kcal, 0.95 g protein, 6.46 g carbohydrate and 3.75 g lipid. Feeding 2b, IMF, comprised per 100 ml 68 kcal, 1.4 g protein, 7.3 g carbohydrate and 3.5 g fat.
Feeding 3a and 3b were energy matched compositions of human milk and IMF.
Feeding 3a, comprised per 100 ml 72 kcal, 1.17 g protein, 8.35 g carbohydrate and 3.60 g fat. Feeding 3b equals feeding 2b.

*Methods:* In total 26 adult male Wistar rats (aged 10 weeks at the start of the experiment) were housed individually. After a 4 h fasting period, 8-10 animals were fed 2 ml of a test composition. Subsequently, blood samples (200 µl) were collected in heparinised chilled tubes at t=0, 5, 10, 15, 30, 60, 90, and 120 minutes after feeding. The experiment was repeated (cross over design) after ate least 1 week rest. Plasma insulin was measured by radioimmunoassay (RIA, of Linco Research) according to the manufacturer's instructions. Plasma glucose was measured with an oxidase-peroxidase method in 96-wells format (Roche Diagnostics, #1448668). In one experiment feeding 1a was compared with feeding 1b. In two other similar experiments feeding 2a was compared to feeding 2b and feeding 3a was compared to feeding 3b, respectively.
*Results:* The post-prandial peak of glucose as well as of insulin was lower in rats fed low protein than in rats fed a meal with a higher protein content. The area under the curve (AUC) of insulin and, to a lesser extent, glucose is lower in rats fed lower protein (Table 1). Decreased levels of both insulin and glucose is indicative for a decreased insulin resistance and/or insensitivity, which is believed contribute to the prevention of central obesity later-in-life. The decreased insulin resistance (or increased insulin sensitivity) was confirmed by the Belfiori formula.
At the same time the AUC of the total amino acids was not significantly lower in rats consuming low protein feedings and the AUC of the essential amino acids (sum of threonine, histidine, lysine, phenylalanine, arginine, tryptophane, leucine, valine, isoleucine and methionine was comparable between feedings tested (data not shown) indicating a sufficient bioavailability of the proteins to ensure proper growth and development.

**Table 1: Effects of proteins on post-prandial peak time, maximal peak height and area under the curve of glucose and insulin.**

| Effect | Feeding 1a | Feeding 1b |
|---|---|---|
| Maximal peak height | | |
| Glucose(mMl ± se) | 10.50±0.67 | 9.92±0.35# |
| Insulin (pM/l ± se) | 491.9±107.6 | 359.9±48.1¶ |
| Sum 15 (± se) | | |
| Glucose (mM/l*15min) | 36.7±1.98 | 35.4±1.05 |
| Insulin (nM/1*15min) | 7.34±1.47 | 6.15±0.67* |
| Sum 30 (± se) | | |
| Glucose (mM/1*30min) | 44.7±1.9 | 43.8±1.3# |
| Insulin (nM/1*30min) | 13.8±1.3 | 12.2±1.3* |
| Sum 120 (± se) | | |
| Glucose (nuM/41*120min) | 69.1±2.4 | 12.09±0.39¶ |
| Insulin (nM/1*120min) | 13.8±1.3 | 12.2±1.3* |
| Belfiore | 1.0157±0.0022 | 1.0191±0.0030* |

| | | |
|---|---|---|
| *: P=0.1 ¶: P≤0.15 #:P=<0.2 | | |

**Table 2: Effects of protein concentration on post-prandial peak time, maximal peak height and area under the curve of glucose and insulin.**

| Feeding | Volume matched | | Energy matched | |
|---|---|---|---|---|
| Effect | 2a | 2b | 3a | 3b |
| Maximal peak height | | | | |
| Glucose(mM ± se) | 6.18±0.13 | 6.69±0.21 | 6.80±0.25 | 6.34±0.12 |
| Insulin (nM ± se) | 1.52±0.13 | 2.03±0.19 | 0.77±0.07 | 1.17±0.12 |
| iAUC 15 (± se) | | | | |
| Glucose (mM* 15min) | 11.9±2.5 | 8.9±3.4 | 7.0±1.5 | 5.8±2.1 |
| Insulin (nM*15min) | 14.5±1.2 | 17.9±1.5# | 4.9±0.7 | 7.5±0.6# |
| iAUC 30 (± se) | | | | |
| Glucose (mM*30min)20.2±4.8 | | 11.7±5.4 | 12.4±2.9 | 9.3±3.9 |
| Insulin (nM*30min) | 24.3±2.3 | 33.3±2.6¥ | 7.6±0.9 | 9.9±0.9* |
| iAUC 120 (± se) | | | | |
| Glucose (mM* 120min) | 81.7±20.8 | 53.0±26.6* | 41.2±12.9 | 41.8±24¶ |
| Insulin (nM*120min) | 37.3±5.3 | 55.9±8.0¥ | 15.1±1.8 | 16.0±1.5 |
| Belfiore | 1.0004 | 1.0003# | 1.0005 | 1.0004¶ |

| | | | | |
|---|---|---|---|---|
| #: P=0.02 ¥: P=0.05 *: P=0.1¶: P=0.2 | | | | |

### Example 2 Composition with lower protein concentration effects body fat distribution in infants as determined by ultrasound measurements

The effect of breastfeeding (low protein, between 0.6 to 1.0 g protein pr 100 ml) versus standard formula feeding (high protein, average of 1.4 g protein per 100 ml) on body fat and fat distribution children. 229 Dutch children were included that underwent an ultrasound examination of the abdomen at the average age of 13 months.
Body fat and fat distribution: All ultrasound examinations of this study were performed with an ATL HDI 5000 (WA, Bothell) with a linear (L 12-5) and a curved transducer (C7-4).
1 Preperitoneal and subcutaneous fat tissue was measured according to the method described by Suzuki et al (Am J Med 1993;95(3):309-14.), with the following adaptations: The change in slope of the different layers to parallel layers served as the apical reference point to measure. The optimal image to measure with regard to midline cut and parallelity of the fat layers was chosen. Preperitoneal fat was measured as areas of between 1 cm and 2 cm length along the midline, starting from the reference point in direction of the navel (PP area). Subcutaneous fat was measured as areas between 1 cm and 2 cm length along the midline starting from the reference point in direction of the navel (SC area). Moreover, ratios of preperitoneal fat and subcutaneous fat were calculated. All measurements were performed off-line.
Bottle feeding, feeding 2, was defined as duration of exclusively breastfeeding equal or smaller than 1 month versus breast feeding. Breast feeding, feeding 1, was defined as more than 4 months exclusively breast feeding. The infants were 13.1±2.2 and 13.1±2.3 months old in feeding group 1 and 2, respectively.
All data were analyzed using ANOVA and the SPSS statistical package (SPSS Inc.®, Chicago, IL Version 11 for Windows).
2 Comparison of abdominal fat and fat distribution in breast fed versus bottle fed infants: After adjustment for maternal pre-pregnancy BMI and educational level, a clear trend toward lower ratio of preperitoneal fat / subcutaneous fat tissue was observed in feeding 1 having low protein levels. See Table 3. The body weights, body length and body mass index (BMI) were unchanged in both feeding groups. See Table 4. This is indicative for the finding that low protein levels in early nutrition can
influence body fat composition and/or distribution in favour of less visceral fat tissue compared to the subcutaneous fat tissue.
Moreover, the described method of measurements of preperitoneal and subcutaneous fat tissue are a suitable method to investigate abdominal fat in groups of children below 48 months of age.

**Table 3: body fat distribution as a result of early nutrition with different protein levels**

| Preperitoneal fat | | Subcutaneous fat | PP/SC | ratio |
|---|---|---|---|---|
| Feeding 1 (n=71) | 0.10 | 0.25 | 0.43 | |
| Feeding 2 (n=65) | 0.11 | 0.23 | 0.56 | p=0.08 |

**Table 4: Body characteristics of infants.**

| Characteristic child | Feeding 1 (n=71) | Feeding 2 (n=65) | p value |
|---|---|---|---|
| SD score current height | -0.04(±0.9) | +0.1(±0.9) | 0.31 |
| SD score current weight | -0.35(±0.9) | -0.33(±0.8) | 0.89 |
| SD score current BMI | -0.38(±1.0) | -0.49(±0.8) | 0.47 |

### Example 3: Blood glucose/insulin and non-digestible oligosaccharides

*Animals and treatment*: Adult male Wistar rats (n=7) were given a galacto-oligosaccharide (GOS) load, cellulose load or water via a gastric canula on day 1. A 6 ml bolus load was administered equal to 50% of their daily fiber intake; GOS was transgalacto-oligosaccharides obtained from Elix'or (Borculo Domo). Fiber was dissolved in water. About 24 h later (on day 2) an oral glucose tolerance test was carried out and the postprandial glucose and insulin course was monitored for 120 min upon the intragastric injection of a carbohydrate load (2 g/kg body weight). To this end blood samples were drawn repeatedly via a jugular vein canula. Intragastric injection of water or a cellulose solution in water on day 1 served as control. As the Elix'or preparation also comprised digestible carbohydrates (mainly lactose), the two control injections were co-administered with carbohydrates to correct for this. *Results:* pre-treatment with GOS clearly decreased the amount of insulin secreted, resulting in significant (p<0.05) lower incremental AUC values. Blood glucose levels were not affected significantly. Pre-treatment with cellulose or water did not modulate the insulin secretion, see Table 5.

**Table 5: Insulin and glucose levels levels in rats.**

| Pre-treatment with: | AUC insulin (pM*30 min) | AUC glucose (mM*30 min) |
|---|---|---|
| Water | 41 ± 7 | 69 ± 10 |
| Cellulose | 46 ± 8 | 75 ± 9 |
| GOS | 22 ± 4 | 74 ± 15 |

### Example 4: Infant formula

Infant nutrition comprising a lipid component providing 48% of the total calories, a protein component providing 8% of the total calories and a digestible carbohydrate component providing 44% of the total calories; (i) the lipid component comprising based on total fatty acids: 10 wt.% LA; 20 wt.% MCFA; 0.2 wt.% DHA, 0.05 wt.% EPA; the LA/ALA ratio is 5.1; (ii) the digestible carbohydrate component comprising 51 gram lactose/100 gram powder; 0.36 g galacto-oligosaccharides with DP 2-6 and 0.4 g fructo-oligosaccharides with DP 7-60; (ii) the protein component comprising cow's milk protein. The label of the package of this infant nutrition indicates that it is used for preventing the development of one or more disorders later-in-life, wherein said disorder is selected from the group consisting of type 2 diabetes, fasting hyperglycaemia, insulin resistance, visceral adiposity, hyperinsulinemia, hypertension, cardiovascular disease, cerebrovascular disease, artherosclerose, dyslipidaemia, hyperuricaemia, fatty liver, osteoarthritis and sleep apnoea.

## Claims

1. Use of a composition comprising a lipid, protein and digestible carbohydrate component wherein the protein component provides less than 9% of the total calories, the lipid component provides 35 to 55% of the total calories and the digestible carbohydrate component provides 30 to 60% of the total calories for the manufacture of a nutritional composition to be administered to a human with the age below 36 months and for
a. preventing and/or treating visceral adiposity;
b. preventing and/or treating the accumulation of visceral fat tissue to an excessive amount; and/or
c. decrease the ratio of visceral fat to subcutaneous fat tissue.

2. Use of a composition comprising a lipid, protein and digestible carbohydrate component wherein the protein component provides less than 9% of the total calories, the lipid component provides 35 to 55% of the total calories and the digestible carbohydrate component provides 30 to 60% of the total calories for the manufacture of a nutritional composition to be administered to a human with the age below 36 months and for preventing visceral adiposity at the age above 36 months, particularly to prevent visceral adiposity at the age above 8 years, particularly above 13 years.

3. Use according to claim 1 for reducing the prevalence of disorders later in life, preferably selected from diabetes and cardiovascular diseases.

4. Use according to any of claim 1, 2 or 3, wherein the composition comprises a protein component providing between 7.2 and 8.0% protein based on calories.

5. Use according to any of the preceding claims, wherein the protein component is derived from cows' milk protein.

6. Use according to any of the preceding claims, wherein the protein is hydrolyzed.

7. Use according to any of the preceding wherein the lipid component comprises (i) linoleic acid (LA) and alpha-linolenic acid (ALA) in a weight ratio of LA/ALA between 2 and 6; (ii) less than 14.5 wt.% LA based on total fatty acids; (iii) long chain polyunsaturated fatty acids (LC-PUFA); and optionally (iv) 3-50 wt.% medium chain fatty acids (MCFA).

8. Use according to any of the preceding claims wherein the composition further comprises non-digestible oligosaccharides.

9. Use according to claim 8, wherein the non-digestible oligosaccharide is at least one component selected from the group consisting of fructo-oligosaccharide, galacto-oligosaccharides and galacturonic acid oligosaccharides

10. Use according to claim 8 or 9 wherein the composition comprises from 0.5 to 10 wt.% non-digestible oligosaccharide based on dry weight of the composition.

11. Use according to any one of the preceding claims, wherein at least 90 wt.% of the digestible carbohydrate is lactose.

12. Use according to any one of the preceding claims, wherein the nutritional composition is administered to a human with the age below 12 months.

## Patentansprüche

1. Verwendung einer Zusammensetzung, die eine Lipidkomponente, eine Proteinkomponente und eine verdauliches Kohlenhydratkomponente umfasst, wobei die Proteinkomponente weniger als 9% der gesamten Kalorien zur Verfügung stellt, die Lipidkomponente 35 bis 55% der gesamten Kalorien zur Verfügung stellt und die verdauliche Kohlenhydratkomponente 30 bis 60% der gesamten Kalorien zur Verfügung stellt, zur Herstellung einer Nährstoffzusammensetzung zur Verabreichung an Menschen mit einem Alter von weniger als 36 Monaten und zur
a. Vorbeugung und/oder Behandlung von viszeraler Adipositas;
b. Vorbeugung und/oder Behandlung von der Ansammlung von Viszeralfettgewebe in einer übermäßigen Menge; und/oder
c. Verringerung des Verhältnisses von Viszeralfett zu subkutanem Fettgewebe.

2. Verwendung einer Zusammensetzung, die eine Lipidkomponente, eine Proteinkomponente und eine verdauliches Kohlenhydratkomponente umfasst, wobei die Proteinkomponente weniger als 9% der gesamten Kalorien zur Verfügung stellt, die Lipidkomponente 35 bis 55% der gesamten Kalorien zur Verfügung stellt und die verdauliche Kohlenhydratkomponente 30 bis 60% der gesamten Kalorien zur Verfügung stellt, zur Herstellung einer Nährstoffzusammensetzung zur Verabreichung an Menschen mit einem Alter von weniger als 36 Monaten und zur Vorbeugung von viszeraler Adipositas im Alter von über 36 Monaten, insbesondere zur Vorbeugung von viszeraler Adipositas in einem Alter von über 8 Jahren, insbesondere über 13 Jahren.

3. Verwendung nach Anspruch 1, zur Verringerung des Auftretens von Erkrankungen im späteren Leben, die insbesondere aus Diabetes und Herz-Kreislauf-Erkrankungen ausgewählt sind.

4. Verwendung nach einem der Ansprüche 1, 2 oder 3, wobei die Zusammensetzung eine Proteinkomponente umfasst, die zwischen 7,2 und 8,0% kalorienbasierte Proteine zur Verfügung stellt.

5. Verwendung nach einem der vorhergehenden Ansprüche, wobei die Proteinkomponente aus Kuhmilchprotein stammt.

6. Verwendung nach einem der vorhergehenden Ansprüche, wobei das Protein hydrolisiert ist.

7. Verwendung nach einem der vorhergehenden Ansprüche, wobei die Lipidkomponente (i) Linolsäure (LA) und alpha-Linolensäure (ALA) in einem Gewichtsverhältnis LA/ALA von 2 bis 6, (ii) weniger als 14,5 Gew.-% LA, bezogen auf die gesamten Fettsäuren, (iii) langkettige polyungesättigte Fettsäuren (LC-PUFA) und gegebenenfalls (iv) 3-50 Gew.-% mittelkettige Fettsäuren (MCFA) umfasst.

8. Verwendung nach einem der vorhergehenden Ansprüche, wobei die Zusammensetzung ferner unverdauliche Oligosaccharide umfasst.

9. Verwendung nach Anspruch 8, wobei das unverdauliche Oligosaccharid mindestens eine Komponente ausgewählt aus der Gruppe bestehend aus Fructo-Oligosacchariden, Galacto-Oligosacchariden und Galacturonsäure-Oligosacchariden ist.

10. Verwendung nach Anspruch 8 oder 9, wobei die Zusammensetzung 0,5 bis 10 Gew.-% unverdauliche Oligosaccharide umfasst, bezogen auf das Trockengewicht der Zusammensetzung.

11. Verwendung nach einem der vorhergehenden Ansprüche, wobei mindestens 90 Gew.-% der verdaulichen Kohlenhydrate Lactose sind.

12. Verwendung nach einem der vorhergehenden Ansprüche, wobei die Nährstoffzusammensetzung einem Menschen im Alter von weniger als 12 Monaten verabreicht wird.

## Revendications

1. Utilisation d'une composition comprenant un composant lipidique, un composant protéinique et un composant glucidique digestible, dans laquelle le composant protéinique fournit moins de 9% de l'ensemble des calories, le composant lipidique fournit de 35 à 55% de l'ensemble des calories et le composant glucidique digestible fournit de 30 à 60% de l'ensemble des calories pour la fabrication d'une composition nutritionnelle destinée à être administrée à un être humain âgé de moins de 36 mois et destinée à :
a. prévenir et/ou traiter l'adiposité viscérale;
b. prévenir et/ou traiter l'accumulation excessive de matières grasses viscérales ; et/ou
c. réduire le taux de matières grasses viscérales en matières grasses sous-cutanées.

2. Utilisation d'une composition comprenant un composant lipidique, un composant protéinique et un composant glucidique digestible, dans laquelle le composant protéinique fournit moins de 9% de l'ensemble des calories, le composant lipidique fournit de 35 à 55% de l'ensemble des calories et le composant glucidique digestible fournit de 30 à 60% de l'ensemble des calories, pour la fabrication d'une composition nutritionnelle destinée à être administrée à un être humain âgé de moins de 36 mois et pour prévenir l'adiposité viscérale à un âge supérieur à 36 mois, notamment pour prévenir l'adiposité viscérale à un âge supérieur à 8 ans, notamment à un âge supérieur à 13 ans.

3. Utilisation selon la revendication 1 pour réduire la prévalence de désordres à un âge plus avancé, de préférence sélectionnés parmi le diabète et les maladies cardiovasculaires.

4. Utilisation selon l'une quelconque des revendications 1, 2 ou 3, dans laquelle la composition comprend un composant protéinique fournissant de 7,2 à 8,0% de protéines exprimé par rapport aux calories.

5. Utilisation selon l'une quelconque des revendications précédentes, dans laquelle le composant protéinique est obtenu à partir de protéines de lait de vache.

6. Utilisation selon l'une quelconque des revendications précédentes, dans laquelle le composant protéinique est hydrolysé.

7. Utilisation selon l'une quelconque des revendications précédentes, dans laquelle le composant lipidique comprend
(i) de l'acide linoléique (LA) et de l'acide alpha-linolénique (ALA) en un rapport pondéral LA/ALA compris entre 2 et 6;
(ii) moins de 14,5 % en poids de LA exprimés par rapport à l'ensemble des acides gras;
(iii) des acides gras polyinsaturés à chaînes longues (LC-PUFA); et éventuellement
(iv) 3-50 % en poids d'acides gras à chaînes moyennement longues (MCFA).

8. Utilisation selon l'une quelconque des revendications précédentes, dans laquelle la composition comprend en outre des oligosaccharides nondigestibles.

9. Utilisation selon la revendication 8, dans laquelle l'oligosaccharide nondigestible est au moins un composant sélectionné dans le groupe consistant en un fructo-oligosaccharide, des galacto-oligosaccharides et des oligosaccharides d'acide galacturonique.

10. Utilisation selon la revendication 8 ou 9, dans laquelle la composition comprend de 0,5 à 10 % en poids d'oligosaccharide nondigestible exprimés par rapport au poids à sec de la composition.

11. Utilisation selon l'une quelconque des revendications précédentes, dans laquelle au moins 90% en poids du glucide digestible est du lactose.

12. Utilisation selon l'une quelconque des revendications précédentes, dans laquelle la composition nutritionnelle est administrée à un être humain dont l'âge est inférieur à 12 mois.
